# EUROPEAN PATENT APPLICATION

(11) **EP 0 828 005 A2**
(43) Date of publication of application: **11.03.1998**
(21) Application number: 97115477.8
(22) Date of filing: 05.09.1997
(51) Int. Cl.: C12N 15/86, A61K 48/00

(54) **Apoptotic agents for tumor cells of the nervous system**

(30) Priority: 06.09.1996 JP 257536/96
(71) Applicant: JCR PHARMACEUTICALS Co., LTD., Ashiya, Hyogo 659 (JP)
(72) Inventor: Chattopadhyay, Subhendra, San Diego, California 92122 (US)
(74) Representative: Lederer, Franz, Dr.

(57) **Abstract**

According to the present invention, there are provided apoptotic agents for tumor cells of the nervous system which comprise a vector having the γ₁34.5 gene deleted from the genome of a herpes simplex virus. The vectors are able to cause infection in tumor cells of the nervous system, thereby inducing apoptosis, and also enable virus derivatives with enhanced thymidine-kinase sensitivity to be prepared, thus opening up a way for utilizing a drug for gene therapy.

## Description

The present invention relates to therapeutic agents and compositions for tumors of the central nervous system, such as glioblastoma. Glioblastoma is difficult to be treated and cured by means of the conventional therapies.

The normal cell death called apoptosis is regarded as a clean, normal cell death in that cells disappear after passing through nuclear condensation, cytoplasmic degeneration and the like, as compared with necrosis being accompanied by inflammation.

In living bodies, the phenomenon of apoptosis takes place in many diverse facets. Typical examples of apoptosis include the normal cell cycle, cell death during development, cell death in response to cellular immunity as is the case with killer T cells or natural killer cells, and cytokine-driven cell death.

In the cranial nervous system, for example, nerve cell death plays an important role in the formation of synapses, whereby 20 to 80 % of the nerve cells fall off and undergo the normal cell death (apoptosis). In the cases of sympathetic ganglion cells (SGC), sensory nerve cells and motor nerve cells, for example, nerve cell death is brought about by removal of the nerve growth factor (NGF), neurotrophic factor, etc. It also has been proven that elimination of the neurotrophic factor causes the gene signal to pass from c-jun to NGF1-A through c-fos, junB, rhl and c-myb to thereby give rise to apoptosis.

Such nerve cell deaths have been confirmed to occur under genetic control, and actually microinjection into cells of the antibodies to the fos family or c-jun leads to significant suppression of apoptosis. Moreover, it is already known that bcl-2, when its expression vector is injected into cells, can inhibit apoptosis. When the bcl-2 oncogene is injected into B cells under the cytokine-free conditions, the B cells do not undergo apoptosis but instead survive for a prolonged period of time [Vaux, D. L., et al, Nature, 335; 440 (1988)].

Some of the nervous-system diseases are caused by degenerative changes in nerve cells or nerve cell death, and their representative examples include Huntington disease and Alzheimer disease. It has been suggested that these diseases are brought about by apoptosis, and there were detected the nerve cells having undergone fragmentation of DNA, or one of the characteristic features of apoptosis [Dragunow, M., et al., Neuroreport, 6: 1053-1057, 1995]. Under these circumstances, there is expected the development of novel therapy and prophylaxis to control apoptosis by utilizing the genes associated with apoptosis or their expression substances. Suppressers and promoters of cell death could provide the therapeutic agents against a variety of diseases.

The gene therapy for brain diseases is a mankind's dream. However, complicated problems are involved in the gene therapy for diseases of the nervous system, in spite of the fact that lots of clinical application trials are under way with the human gene therapy against adenosine deaminase deficiency, familial hypercholesterolemia, and the like. (Suhr, S. T. & Gage, F. H.: Arch. Neurol., 50: 1252-1268, 1993). Gene therapy for the central nervous system is rendered difficult, for example, by interactions of the nervous system compartments with nerve cells and the blood-brain barrier.

As an approach toward the gene therapy in the central nervous system, there are currently attempted a method which involves using a virus as a gene carrier to directly incorporate a gene into the nervous-system cells, and a method which comprises introducing a gene in cultured cells, followed by transplanting into a targeted site.

In addition to apoptosis in the nervous system, it has also been clarified that the phenomenon of apoptosis is induced by death of cancer cells brought about by the stimuli of anti-cancer agents. A large number of drugs, which act through individually different mechanisms, cause cancer cells to undergo similar apoptoses, and the process of genetic control of cells is therefore considered to be implicated in the cancer cell death, as well [Borner, M.M. et al., Cancer Res. 55: 2122-2128, 1995].

Referring to the chemotherapy of cancers, topoisomerase inhibitors, alkylating agents, etc. have been found to act to induce apoptosis

Any cells, after having suffered from disturbances in the genetic control process of apoptosis, turn into apoptosis-resistant cells, and bcl-2 and bcl-XL which suppress apoptosis have been isolated in large quantities from the cancer cells having acquired such drug resistance. It is also known that infection of bacteria with viruses results in induced apoptosis or enhanced susceptibility to apoptosis in the infected cells, which may be exemplified by apoptosis of T cells after being infected with HIV. On the other hand, such viral species as HSV, EBV, adenovirus and baculo virus have been confirmed to allow incorporation of the genes capable of inhibiting apoptosis in hosts.

Such facts suggest a possibility that drugs for the treatment of cancers and tumors will be developed by making use of apoptosis.

One of the tumors peculiar to the central nervous system is known as glioblastoma, the most common tumor in human brains, which is normally fatal [Levine A, et al., Cancer: Principles and Practice of Oncology, 2: 1557 (1987)]. In the U.S.A., there are reported about 5,000 per annum of adult patients with brain tumors, not less than 30 % of which is accounted for by the ones with glioblastoma, but there has been known so far no effective treatment method for such brain tumors. Glioblastoma, when it reaches about 100 g in weight within the cranial space, becomes lethal [Shapiro, W. R., Seminars in Oncology, 71: 38 (1986)].

Expression or transfer into the nervous system cells of anti-tumor genes or anti-tumor substances by means of a safe procedure for the purpose of treatment of such brain tumors is expected to confer the clinical advantages, and eventually will provide therapies against the disorders which have conventionally been considered refractory.

In recent years, the treatment of human brain tumors is being attempted by utilizing genetic engineering to transfer genetic materials into patient's brain cells. Among them, for example, mention can be made of a method which involves administering to a patient an anti-brain-tumor agent, which is in turn activated into an effective form of the drug to attain proliferation inhibition against such tumor cells. Also, there is proposed an approach of incorporating a gene coding for a metabolic enzyme which is absent in humans to thereby produce its pharmacological effects within the targeted cells alone, without causing any reaction with any humans' proprietary metabolic enzymes, whereby no enzymatic metabolism takes place in non-incorporated cells, resulting in freedom from toxicity. When a viral vector is used for such gene incorporation, the method is referred to as VDEPT (which stands for "virally directed enzyme prodrug therapy") [Gutierrez, A. A. et al., The Lancet, 339: 715 (1992)].

The enzyme genes often tested in gene therapy include, for example, herpes simplex virus thymidine kinase (HSV-tk), cytosine deaminase (CD) originated from Escherichia coli and varicella zoster virus thymidine kinase (VZV-tk). By giving a virus capable of expressing such enzyme gene to a patient for infection or by transplanting cells producing such virus into a tumor site, attempts are being made to allow expression of the metabolic enzyme specifically in tumor cells or to directly inject such enzyme gene locally in tumor cells. By incorporation of the enzyme genes, the resultant genetic products or reaction products can act to inhibit proliferation of tumor cells.

Herpes simplex virus type 1 (HSV-1) can be utilized as a vector for incorporating foreign genes into cells of the peripheral and central nervous systems [Friedman, T., Science, 244: 1275 (1989)], and such herpes simplex virus itself may be usable as a gene transfer vector, as well. HSV-1 is a double-stranded DNA virus and is capable of keeping latency in non-dividing neuron cells [Stevens, J. G., et al., Immunol., 70: 31 (1975)]. Herpes simplex type 2 (HSV-2), which infects the genitalia, is employable as a vector to incorporate foreign genes into cells of the genitalia.

One of the major distinctive features of HSV-1 is that the virus can be taken up by, or infect, the peripheral nerves to thereby move from the sensory and motor neurons via the synapses to the spinal chord, brain stem, cerebellum and cerebrum, etc. [Koprowski, H., Persistent Viruses, pp. 691, Academic Press, N.Y., U.S.A. (1978)].

Because of this, the HSV-1 vector can allow administration at sites being far remote from the target tissue, such as intravenous injection, in addition to direct injection into the target cells, and can be said to be effective as a drug for gene therapy and its processed, formulated preparations.

The HSV-1 vector can proliferate within appropriate host cells. At the same time, the use of HSV-1 itself tends to damage host animals and actually causes encephalitis in humans, and consequently, its utilization as a drug for gene therapy has fairly severe limitations.

Culver et al., using fibroblast [Culver, K. W. et al., Science, 256: 1550 (1992)], generated the transformed cells, or the HSV-TK producer cells, by transfecting into fibroblast cells a retroviral vector having the herpes simplex virus (HSV-1) thymidine kinase gene (TK) incorporated therein, whereby such transformed cells were termed "packaging cells". Such packaging cells can be prepared easily in accordance with the conventionally known technique.

When the packaging cells of the HSV-TK producing cells are injected into a mouse having a glioblastoma developed in the brain, the vector having the TK gene incorporated therein brings about infection through lysis only in the actively dividing brain tumor cells; then, such dividing tumor cells through uptake of the TK gene develop sensitivity toward the therapy with acyclovir or gancicrovir (GCV) preparations. In the animal experiment with rats, the tumor regression was observed in about 80 % of the animal subjects. [Culver, K.W., Proceedings of the 2nd Brain Tumor Conference of Japan, pp.41 (1993), Zvi Ram et al., Cancer Res. 53:83 (1993)].

The factor behind the finding that VDEPT with HSV-TK and GCV enhances the therapeutic effects against brain tumor in rats is that retrovirus proliferates in the dividing tumor cells but does not infect the non-dividing normal neurons.

However, such VDEPT is found to produce lethal effects even in the surrounding cells which have not been transduced with HSV-TK, and this phenomenon is called and known as "bystander effect"

Acyclovir, after being phosphorylated by virus-specific thymidine kinase, is transformed into its active triphosphate derivative, which is then taken up by a virus to inhibit the DNA synthesis by a viral DNA polymerase, thereby suppressing proliferation of such DNA virus. The above cascade reactions are characterized in that the reactions are not catalyzed by normal cell thymidine kinase and are specifically peculiar to herpes simplex thymidine kinase. Likewise, there is also employed 9-{[2-hydroxy-1-(hydroxymethyl)ethoxy]methyl}-guanine called ganciclovir (GCV), which is a guanosine-derivative based anti-viral agent. GCV is not activated by human thymidine kinase but is phosphorylated effectively by herpes simplex thymidine kinase, leading to inhibition of its DNA synthesis [Field, A.K. et al., Proc. Natl. Acad. Sci, USA, 80: 4139 (1983)].

With increasing enzymatic activity of HSV-tk, more enhanced therapeutic effect is expected from it, and attempts were therefore made to construct HSV-TK mutants, with the result that there have been obtained HSV-tk mutants showing increased GCV phosphorylating activity as compared with the wild type one [Munir, K. M. et al., Proc. natl. Acad. Sci USA, 99: 4012 (1993)].

By constructing TK-gene deficient mutants of HSV-1, followed by injection without administration of any other medicaments, attempts were made to take advantage of neurovirulence or neurotoxicity originated from the γ₁34.5 gene of such mutant virus to thereby induce tumor cell death [Artuza, R. L. et al., Science, 252: 854 (1991)]. Such TK-gene deficient HSV-1, which has 360 base pairs of the TK gene deleted, lacks TK activity, and consequently, such mutant virus proliferates rapidly only in the dividing cells of the central nervous system, without replicating in non-dividing cells. In these research studies, the suppression of nude mouse tumors has been observed.

Several reports were published on the discovery and characterization of the γ₁34.5 gene of HSV-1 which produces neurovirulence [Chou and Roizman, J. virol., 57: 629 (1986) and Ackermann et al., J. Virol. 58: 843 (1986)]. The γ₁34.5 gene codes for a protein consisting of 263 amino acid residues, and a mutant virus lacking in the γ₁34.5 gene loses the capability to proliferate in the central nervous system consisting of non-dividing cells.

The method for isolating HSV-1 has already been known [Ejercito, P.M.et al, J. Gen. Virol. 2: 357 (1968)], while it is also well known to construct the genetically engineered, recombinant viruses, R3616 and HSV-1(R) [Chou, J. et al., Science, 250: 1262 (1990)]. R3616 is a mutant virus having one kilo base pairs deleted from the DNA sequence containing the γ₁34.5 gene, wherein the range of said one kilo base pairs is encompassed and defined by a location corresponding to the 28th amino acid moiety cleaved from the γ₁34.5 gene with the restriction enzyme BstEII and the 3' terminus is cleaved from the gene with the restriction enzyme Stu I. HSV(R) is a reconstructed virus generated by reincorporating the γ₁34.5 gene into R3616.

At present, there is known none of safe apoptotic agents for tumors of the nervous system which contain a virus itself.

The present inventor, after extensive research work, found that a mutant type herpes simplex virus (HSV-1-d-34.5), which is R3616, exhibits attenuated neurovirulence as well as protein-synthesis termination in infected cells, while demonstrating additionally sensitivity toward acyclovir, and thus can offer the advantage of enhanced efficacy secured in clinical use.

The present invention relates to apoptotic agents for tumor cells of the nervous system which comprise a vector having the γ₁34.5 gene deleted from the genome of a herpes simplex virus.

The viral vector to be used in this invention, or the vector having the γ₁34.5 gene deleted from the genome of a herpes simplex virus, which may be exemplified by R3616, is free from neurovirulence to hosts and after infection, can proliferate only in the glioma cells, or the dividing brain cells, to bring about apoptosis. The virus (HSV-1-d-34.5) being transformed by genetic engineering can be utilized to destroy human glioblastoma cells. The procedure of constructing R3616 was described by Perg et al. [Perg, G. et al., J. Viol. 69: 3033 (1995)]. In the same manner, the γ₁34.5 gene can be deleted from the genome of other herpes simplex virus.

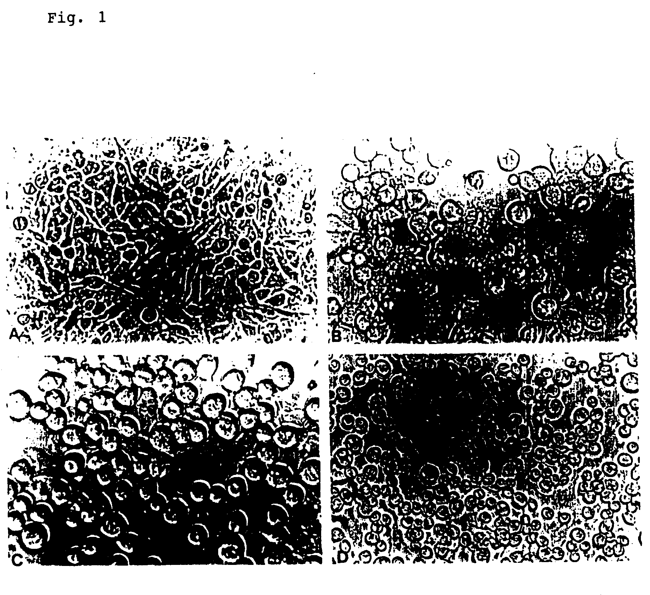

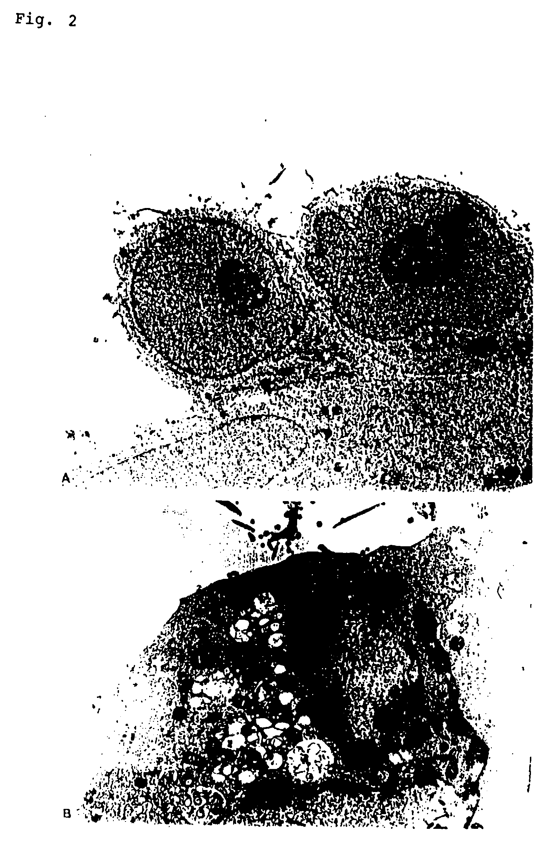

The brain tumor cells are generally surrounded by the differentiated neuron cells and differentiation-terminated gliacytes. Using as a tumor cell the human glioma cell lines, U-251MG (glioblastoma multiform) and U-87MG (anaplastic glioma: ATCC), with astrocytes, microglia and central nervous system neurons being used as a normal gliacyte, the present inventor conducted morphological studies by means of a light microscope and electron microscope (Figs. 1 and 2).

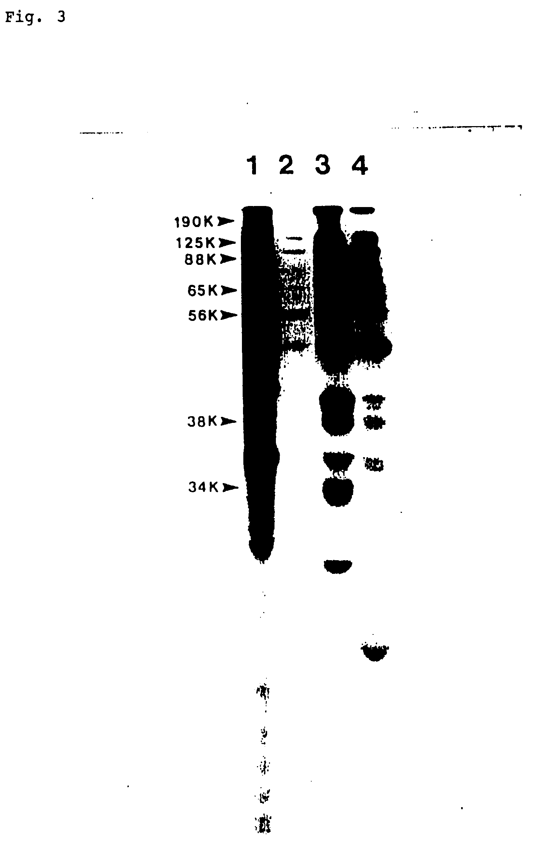

Investigation was also carried out into the host protein synthesis in glioma cells after being infected with a genetically engineered recombinant virus (R3616) (Fig. 3): glioma cells and normal cells were infected with R3616 and the wild type virus for 2 hours, respectively. After the infection, the cells were labeled with ³⁵S-methionine (50 uCi/ml). The infected cells as well as uninfected cells were harvested, solubilized and electrophoresed on SDS polyacrylamide gel. The results of these experiments indicated that the mutant type virus (R3616) is able to induce apoptosis in glioma cells. Actually, the wild type virus did not produce apoptosis in the presence of the γ₁34.5 gene, whereas the mutant type virus (HSV-1-d-34.5) showed termination of protein synthesis and also was microscopically observed to have undergone apoptosis.

The viruses resort to the function of the γ₁34.5 gene for their capability to replicate and proliferate in the central nervous system. The mutant type herpes viruses which are deficient in this gene, such as R3616, cannot proliferate in the normal central nervous system and are free from toxicities or virulences [Chou, J. et al., Science, 250; 1212 (1990)]. Consequently, it follows that the apoptotic agents of the present invention, if endowed with the capability to target the tumor cells in the central nervous system, could provide a potent therapeutic agent against the disease of glioblastoma being refractory to the conventional treatments. The mutant type virus, R3616, can be admixed and formulated with conventionally known, appropriate stabilizing agents, such as human serum albumin, inorganic salts, amino acids, etc., to be used as a formulated, processed preparation.

Referring to the suggested method of administration, the formulated preparations containing the vectors according to the present invention can be given directly to the targeted cell or tissue, and alternatively, advantage can be taken of the characteristic feature of the herpes simplex virus vectors to inject such formulated preparation at remote sites being far away from the targeted one, wherein intravenous application can be made, as well. Furthermore, there can be employed a method of administration which comprises transplanting at the site of a tumor transformed cells prepared by transfecting such known cells as fibroblasts with R3616.

The preferred dosage amount is at a ratio of not less than 10 vectors per a tumor cell.

R3616 provides a possibility of directly utilizing its characteristic feature of the apoptosis-inducing property and can additionally be used to prepare a mutant type virus derivative (TK-proficient-HSV-1-d-34.5) with enhanced sensitivity toward thymidine kinase, followed by processing into dosage forms of formulated preparations, thereby finding further widened applicability of herpes simplex virus vectors as a drug for gene therapy.

Such virus derivatives exhibit their own proprietary apoptosis-inducing property and furthermore, can permit anti-viral agents to be administered from the outside, thus possibly finding extremely advantageous application in the clinical aspects.

Below described are the examples to illustrate the present invention in more detail, referring to the attached drawings wherein:
Fig. 1 shows the micrographs of cells of U-87MG, a human glioma cell line, (anaplastic glioma; ATCC), which were taken on an optical microscope 10 hours after being infected with the wild type virus (HSV-1), a mutant type virus R3616 (HSV-1-d-34.5) and a reconstructed virus HSV-1(R) as described in Example 2.
   Photograph A: Control cells not infected with any virus; B: Cells infected with a mutant type virus R3616; C: Cells infected with a reconstructed virus HSV-1 (R); D: Cells infected with the wild type virus HSV-1.
Fig. 2 shows the micrographs of U-87MG cells taken on a transmitting electron microscope, as mentioned in Example 2: A: Control cell not infected with any virus; B: Cell infected with a mutant type virus R3616, in which typical nuclear condensation is observed around the periphery of the nuclear membrane.
Fig. 3 is a photograph taken by exposing to an X-ray film the gel yielded in SDS polyacrylamide gel electrophoresis effected in Example 3:
   Lane 1: Cells not infected with any virus; Lane 2: Cells infected with a mutant type virus R3616; Lane 3: Cells infected with a reconstructed virus HSV-1 (R); Lane 4: Cells infected with the wild type virus HSV-1.

### Example 1

In order to determine the applicability in the central nervous system, R3616, a mutant type herpes simplex virus (HSV-1-d-34.5), was evaluated for toxicological effects on animals. Referring to the method of administering specimens, the wild type KOS strain (2 µl) and a mutant type virus R3616 as well as a culture solution as a control were injected directly into the cerebra of rats by use of a gauge needle, respectively, followed by daily observation of the rats for survival and behavior over the 2-weeks period. The rats used were divided into groups each consisting of ten animals, while the virus was dosed to each rat at a rate of 200,000 plaque forming units (PFU) per head as diluted with Eagle's essential minimum medium containing salts as well as 10 % bovine serum and penicillin. The results are shown in Table 1.

**Table 1**

| The number of rats survived after intracerebral injection of the viruses and the found LD₅₀ values | | | |
|---|---|---|---|
| Virus injected (gene type) | Day 3 | Day 14 | PFU/ LD₅₀ |
| HSV-1 (wild type) | 10/10 | 1/10 | 400 |
| R3616 (γ₁34.5-deficient) | 10/10 | 9/10 | >1,000,000 |
| Control | 10/10 | 9/10 | |

Comparison of the LD₅₀ values found after intracerebral injection reveals that a mutant type strain R3616, showing more than 2,000-fold lowered toxicity than the wild type HSV-1, is the viral vector with a remarkably enhanced degree of safety to the normal tissues and cells.

### Example 2

Investigation was carried out into apoptosis of human glioma cells to be induced by the mutant type virus strain R3616.

Cells of U-87G or U-251MG, both of which are the established human glioma cell lines, were suspended at a concentration of 1 x 10⁵/cm² in a medium (DMEM, supplied by Dai-Nippon Pharmaceuticals Co. of Japan) containing 10 % fecal bovine serum, and the resultant suspension was seeded into 6-cm Petri dishes, followed by incubation in a CO₂ incubator at 37°C for 4 to 6 days. After it was confirmed that the cells grew in the whole bottom of each Petri dish, the culture medium was removed by suction, followed by washing with phosphate buffered saline.

A herpes simplex virus mutant strain (R3616), a strain HSV-1(R) as reconstructed by reinserting the γ₁34.5 gene of HSV-1, and the wild type strain (HSV-1), after being diluted with phosphate buffered saline, were used to allow infection at a rate of 10 viral particles per cell, followed by microscopic observation 10 hours later.

Generally, apoptosis of cells proceeds with accompaniment of the cell contraction and chromatin concentration, which are the morphological changes characteristic to apoptosis. With a length of time elapsed after the infection, only the cells infected with the mutant type virus R3616 exhibited the morphological changes peculiar to apoptosis, whereas no morphological changes were observed in the control of non-infected cells, demonstrating that R3616 induced apoptosis (Fig. 1). Observation with a transmitting electron microscope indicated that there was a partial increase (increases in the chromatin moiety) in electron densities within the nucleus (Fig. 2).

### Example 3

A neurovirulence-free virus strain R3616, a strain HSV-1(R) having the γ₁34.5 gene reinserted therein and the wild type strain, in the form of a viral solution, were added individually to cells of the established glioma cell line to allow adsorption at a rate of 10 viruses per cell of for 2 hours. After washing with a phosphate buffered saline solution, cultivation was effected in Eagle MEM (supplied by Dai-Nippon Pharmaceuticals Co. of Japan) containing 2 % fecal bovine serum. Ten hours later, the culture media were replaced by two Eagle MEMs (supplied by Dai-Nippon Pharmaceuticals Co. of Japan) individually containing 50 uCi/ml of ³⁵S-methionine (Amersham) and 1 % fecal bovine serum dialyzed against distilled water but being free from the methionine. For each virus strain, cultivation was conducted in two types of culture media for 2 hours, and the cells were washed and molten in a 100mM Tris-HCl buffer containing 0.1 % SDS. The molten solutions were fractionated by SDS polyacrylamide gel electrophoresis, and the gels were immersed in individual sensitizer solutions manufactured by Amersham Co., followed by drying and exposure to X-ray films (Fig. 3). It was revealed that in the glioma cells infected with the mutant virus strain R3616, negligible intake of ³⁵S-methionine took place and the protein synthesis was remarkably inhibited.

## Claims

1. An apoptotic agent for a tumor cell of the nervous system which comprises a vector having the γ₁34.5 gene deleted from the genome of a herpes simplex virus.

2. An apoptotic agent as claimed in claim 1, wherein the tumor cell of the nervous system is glioblastoma.

3. An apoptotic agent as claimed in claim 1 or 2, wherein the herpes simplex virus is type 1 or type 2.

4. An apoptotic agent as claimed in any of claims 1-3, wherein the said apoptotic agent is processed into the dosage form of an injectable solution.
